# EUROPEAN PATENT APPLICATION

(11) **EP 2 765 126 A1**
(43) Date of publication of application: **13.08.2014**
(21) Application number: 13154199.7
(22) Date of filing: 06.02.2013
(51) Int. Cl.: C07C 29/17

(54) **Hydrogenation of citral in a solvent**

(71) Applicant: DSM IP Assets B.V., 6411 TE Heerlen (NL)
(72) Inventor: The designation of the inventor has not yet been filed
(74) Representative: Kurt, Manfred

(57) **Abstract**

The present invention is concerned with a novel and improved process for the manufacture of tetrahydrogeraniol by hydrogenation of citral in a solvent.

## Description

The present invention is related to a novel and improved process for the manufacture of tetrahydrogeraniol by hydrogenation of citral.

Tetrahydrogeraniol (compound of (II)) is an important and valuable compound for the use in the field of flavour and fragrance applications.

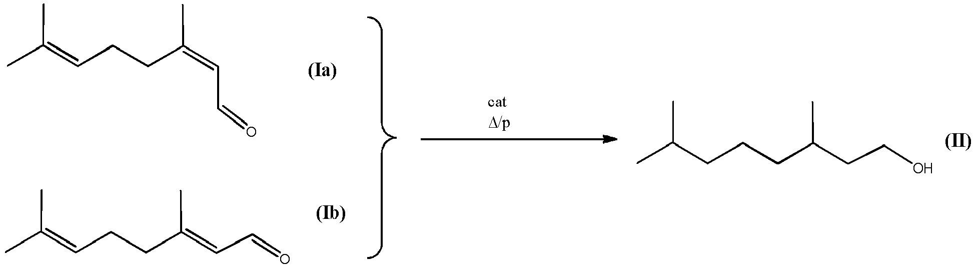

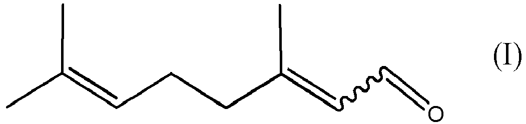

When citral (compound of formula (I)), which is a mixture of neral (compound of formula (Ia)) and geranial (compound of formula (Ib)), is hydrogenated usually a mixture of various reaction products is obtained.

For example the following side products (SP1) - (SP6) 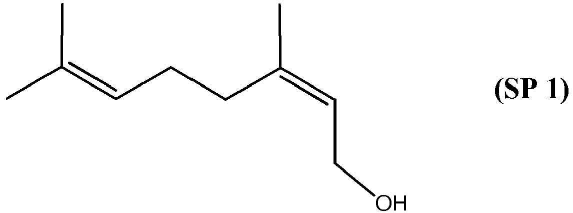 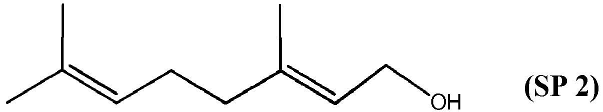 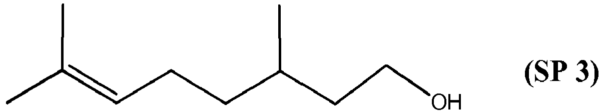 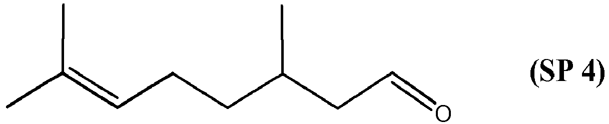 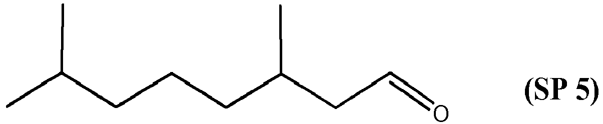 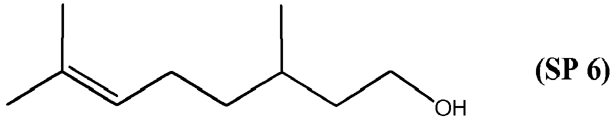 are obtained.

These side products are obtained in various amounts and they are to be removed from the reaction solution at the end of the hydrogenation process to obtain the desired tetrahydrogeraniol (THGOL) in a pure form.
Such purification can be difficult and it involves additional steps and as a consequence thereof the yield of the pure THGOL is not very high.

In the prior art (Devred et al, App. Catal. A, 2009, 356, 154 - 161) the hydrogenation of citral using specific nickel catalyst in methanol (CH₃OH) is described. The yields of this process are not satisfying (which can also be seen in our comparison examples).
Therefore the goal of the present invention was to find an improved hydrogenation of citral to tetrahydrogeraniol whereby the tetrahydrogeraniol is obtained in an excellent yield (with a very low amount of side products) and wherein the catalyst used in that hydrogenation is easy to obtain and easy to be reused.

Surprisingly, it was found that by using a nickel catalyst and a solvent, tetrahydrogeraniol is obtained in an excellent yield and selectivity. Furthermore the catalyst is easy to recycle and to be reused.

Therefore the present invention relates to a process for the production of tetrahydrogeraniol (compound of formula (II)) 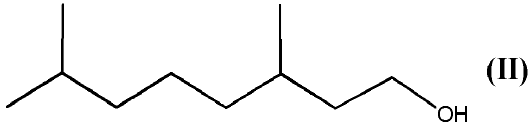 by hydrogenation of citral (compound of formula (I)) 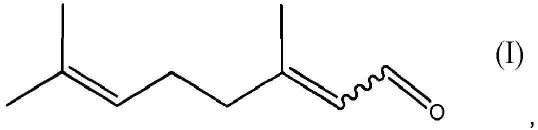 wherein
(i) the hydrogenation is carried out in a solvent or a mixture of solvents,
(ii) a nickel catalyst is used, and
(iii) with the proviso that the solvent or the mixture of solvents does not comprise CH₃OH.

Usually the hydrogenation is carried out by using H₂-gas.
It is possible (and preferred) to use pure H₂-gas, but it would also be possible to use a gas mixture which comprises H₂.

Therefore the present invention relates to a process as described above wherein the hydrogenation is carried out by (pure) H₂-gas.

The hydrogenation is usually carried out under pressure. The pressure is preferably at least 1.5 bar. Usually not more than 50 bar is used. A preferred range for the pressure is 1.5 - 40 bar, more preferably 2 - 30 bar.
The hydrogenation is usually carried out in a vessel, which is suitable to enduring the pressure.

Therefore the present invention relates to a process as described above wherein the hydrogenation is carried out at a pressure of 1.5 - 40 bar, preferably 2 - 30 bar.

The hydrogenation is usually carried out at temperature from 20°C to 120°C. Preferably 40°C - 100°C.

Therefore the present invention relates to a process as described above wherein the hydrogenation is carried out at a temperature of 20°C to 120°C, preferably 40°C - 100°C.

The ratio (related to the weight) of citral to the catalyst in the hydrogenation reaction mixture is usually at least 5:1.

A solvent or a mixture of solvents is used for the hydrogenation. This solvent or solvent mixture does not comprise methanol. That means it is essentially free of methanol.
Methanol is excluded from the scope of the present patent application. As stated above, in the prior art (Devered et al, App. Catal.. A, 2009, 356, 154-161) methanol has been used as a solvent for a hydrogenation (at 2mPa, 40°C, using 300 mg nickel catalyst). But the yield of THGOL using these reaction conditions is not sufficient (less than 15% after 8 hours and less than 80% after 45h).

Therefore it was surprising that when using other solvents the yield (and the conversion) was improved significantly.
Suitable solvents are linear, branched or cyclic aliphatic hydrocarbons having 5 - 10 carbon atoms; aromatic hydrocarbons having 5 - 10 carbon atoms; esters; ethers; alcohols (with the exception of methanol).
It is preferred that solvents are used which are liquid under normal conditions, which allows an easy handling.
Preferred solvents are THF, n-hexane, 2-propanol, toluene and ethyl acetate

Citral (compound of formula (I)), which CAS number is 5392-40-5 and which IUPAC name is 3,7-dimethyl-2,6-octadienal, is commercially available.
As stated above citral is a mixture of the cis-isomer and the trans isomer of 3,7-dimethyl-2,6-octadienal. For the hydrogenation of the present invention the citral, which was used had a ratio of the cis-isomer and the trans isomer of about 60:40 up to about 40:60.

The nickel catalyst used in the process according to the present invention is a heterogeneous catalyst.
Preferred catalysts are nickel alloy catalysts. Such catalysts are also known as "skeletal catalysts" or "sponge-metal catalysts".
Such catalysts are commercially available for example under the tradename Actimet^{®} from BASF (i.e. Actimet M) or under the product name B 111 W, B 112, B 113 W, B 113 Z from Evonik or JM A4000, JM A40A9, JM A2000 from Johnson Matthey Catalysts or Acticat ^{®} from CatAlloy (i.e. Acticat 1000, Acticat 1100, Acticat 1200, Acticat 1600).

Another advantage of the process according to the present invention is that the catalyst can be reused for further hydrogenation.
The catalyst can also be easily recycled.
Usually the catalyst can be used without further treatment. So it is possible to run the hydrogenation batch-wise or continuously.

The following examples serve to illustrate the invention. If not otherwise stated all parts are given are related to the weight and the temperature is given in °C

### Examples

### Example 1 - 2i

All of the hydrogenations have been carried out according as described below. The temperature, pressure, solvent and catalyst are listed in the table: The nickel catalyst (300 mg) was added to a 100 ml reactor fitted with a gas entrainment stirrer. The catalyst was washed with 3x anhydrous ethanol and 2x the reaction solvent. Citral (3 g) and the solvent (30 g) were added and the autoclave was sealed. The reactor was purged 5 times with nitrogen and 5 times with hydrogen. The reactor was heated to the desired temperature and then pressurized to the desired pressure. The reaction mixture was stirred for 20 hours or until no further hydrogen uptake was observed. At the end of the reaction the reactor was cooled to room temperature, the pressure released and purged once with nitrogen. The reaction mixture was filtered and analysed by GC for conversion and selectivity.

In Table 1, three different commercially available nickel catalysts have been tested. All of them do show the same excellent results in regard to conversion (conv.) and yield of THGOL.

**Table 1: Different commercially available nickel catalyst**

| **Exp** | **Catalyst** | **Solvent** | **T (°C)** | **P (bar)** | **Conv. (%)** | **THGOL (%)** |
|---|---|---|---|---|---|---|
| **1a** | Evon i k B 111 W | EtOAc | 80 | 20 | >99 | 99 |
| **1b** | Evon i k B 113 W | EtOAc | 80 | 20 | >99 | 99 |
| **1c** | JM A4000 | EtOAc | 80 | 20 | >99 | 99 |

In a second series, always the same catalyst has been used, but the solvent, the temperature and the pressure has been varied.

The results are summarized in Table 2.

**Table 2**

| **Exp.** | **Catalyst** | **Solvent** | **T (°C)** | **P (bar)** | **Conv. (%)** | **THGOL (%)** |
|---|---|---|---|---|---|---|
| **2a** | JM A4000 | EtOAc | 80 | 20 | >99 | 99 |
| **2b** | JM A4000 | EtOAc | 80 | 10 | >99 | 94 |
| **2c** | JM A4000 | EtOAc | 80 | 5 | >99 | 82 |
| **2d** | JM A4000 | EtOAc | 60 | 10 | >99 | 82 |
| **2e** | JM A4000 | EtOAc | 40 | 20 | 99 | 95 |
| **2f** | JM A4000 | THF | 80 | 10 | 99 | 70 |
| **2g** | JM A4000 | n-Hexane | 80 | 10 | 99 | 87 |
| **2h** | JM A4000 | 2-propanol | 80 | 10 | 99 | 96 |
| **2i** | JM A4000 | Toluene | 80 | 10 | 99 | 87 |

### Example 3 (Comparison Example with a different type of catalyst)

Hydrogenation was carried according to the previous examples (using the same reaction conditions), but using supported precious metal catalysts instead of the nickel catalyst. The following catalysts, which are very common catalysts and are commercially available, were used:
5% Palladium on Carbon;
5% Platinum on Carbon;
2.5% Palladium and 2.5% Platinum on Carbon;
5% Ruthenium and 0.5% Palladium on Carbon.

For all these catalyst the yield of THGOL was never more than 4 %. It was surprising that when using these catalysts the hydrogenation of citral occurred but only minor amounts of THGOL were formed. The main products of the reaction were the side products (SP1) - (SP6).

### Example 4 (Prior art Example from Devred et al, App. Catal. A, 2009 356, 154)

The nickel catalyst (Evonik B 113 Z, 300 mg) was added to a 100 ml reactor fitted with a gas entrainment stirrer. The catalyst was washed with 3x anhydrous ethanol and 2x the reaction solvent. Citral (5 g) and the solvent (40 ml) were added and the autoclave was sealed. The reactor was purged 5 times with nitrogen and 5 times with hydrogen. The reactor was heated to the 40 °C and then pressurized to 20 bar. After a reaction time of 8 hours, a conversion rate of more than 99% and a selectivity of THGOL of 14.1 % was obtained. This example was repeated several times.

### Example 5 (recycling tests)

The following examples serve to demonstrate that these catalyst when used in a process according the present patent application, they show excellent recycling properties.

The hydrogenation and the recycling was carried as follows:

The nickel catalyst (7.5 g, Evonik B 111 W) was placed in 1 litre steel reactor fitted with a gas entrainment stirrer. The catalyst was washed with 2x anhydrous ethanol (30-50 ml), 3x ethyl acetate (30-50 ml). Citral (50 g) and ethyl acetate (500 g) were added and the reactor sealed. The reactor was purged 3 times with nitrogen (5 bar) under stirring (500 rpm) and then 3 times with hydrogen (5 bar) without stirring. The reactor was heated to 80 °C under stirring (500 rpm) and then pressurized to 20 bar. The reaction was stirred (1000 rpm) until consumption of hydrogen was complete. The reactor was cooled to room temperature with stirring (500 rpm), the pressure was released and the reactor was purged once with nitrogen (5 bar). The stirring was stopped and the catalyst was allowed to settle. The product was removed from the reactor via a "dip tube" containing a sinter filter. Additional citral (50 g) and ethyl acetate (500 g) were added through the same dip tube and the reaction was described above was repeated.

**Table 3**

| **Run** | **Temp. (°C)** | **Pressure (bar)** | **Conv. (%)** | **THGOL (%)** |
|---|---|---|---|---|
| **Initial Experiment** | 80 | 20 | 99 | 91 |
| **Recycle 1** | 80 | 20 | 99 | 91 |
| **Recycle 2** | 80 | 20 | 99 | 91 |
| **Recycle 3** | 80 | 20 | 99 | 90 |
| **Recycle 4** | 80 | 20 | 99 | 91 |
| **Recycle 5** | 80 | 20 | 99 | 99 |

It can be seen even after the 5^{th} recycling run the conversion and the yield was still excellent.

## Claims

1. A process for the production of a compound of formula (II) 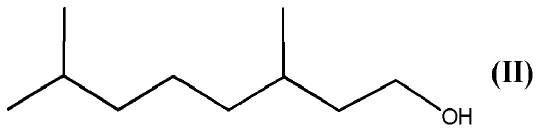 by hydrogenation of a compound of formula (I) 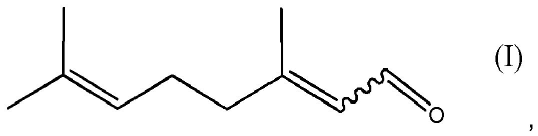 wherein
(i) the hydrogenation is carried out in a solvent or a mixture of solvents,
(ii) a nickel catalyst is used, and
(iii) with the proviso that the solvent or the mixture of solvents does not comprise CH₃OH.

2. Process according to claim 1, wherein the hydrogenation is carried out by using pure H₂-gas or a gas mixture which comprises H₂.

3. Process according to anyone of the preceding claims, wherein the hydrogenation is usually carried out under pressure of at least 1.5 bar. preferably at a pressure of 1.5 - 40 bar, more preferably 2 - 30 bar.

4. Process according to anyone of the preceding claims, wherein the hydrogenation is carried out at temperature from 20°C to 120°C, preferably 40°C - 100°C.

5. Process according to anyone of the preceding claims, wherein the hydrogenation is carried out in at least one solvent chosen from the group consisting of linear, branched or cyclic aliphatic hydrocarbons having 5 - 10 carbon atoms; aromatic hydrocarbons having 5 - 10 carbon atoms; esters; ethers; alcohols.

6. Process according to anyone of the preceding claims, wherein the hydrogenation, is carried out in THF, n-hexane, 2-propanol, toluene or ethyl acetate.

7. Process according to anyone of the preceding claims, wherein the hydrogenation is carried out in ethyl acetate

8. Process according to anyone of the preceding claims, wherein the catalyst is a heterogeneous catalyst.

9. Process according to anyone of the preceding claims, wherein the catalyst is a nickel alloy catalyst.

10. Process according to anyone of the preceding claims, wherein the hydrogenation is carried out batch-wise or continuously.
